(19) Europäisches Patentamt / European Patent Office / Office européen des brevets

(11) **EP 3 906 408 B1**

(12) **EUROPEAN PATENT SPECIFICATION**

(45) Date of publication and mention
of the grant of the patent:
**18.10.2023 Bulletin 2023/42**

(21) Application number: **19836593.4**

(22) Date of filing: **31.12.2019**

(51) International Patent Classification (IPC):
**G01N 33/00** (2006.01)   **G01N 22/04** (2006.01)
**A01G 25/16** (2006.01)

(52) Cooperative Patent Classification (CPC):
**G01N 33/0098; A01G 25/16; G01N 22/04;**
A01G 25/09; A01G 27/003

(86) International application number:
**PCT/IB2019/061469**

(87) International publication number:
**WO 2020/141456 (09.07.2020 Gazette 2020/28)**

(54) **DEVICE AND AGRICULTURAL DEVICE FOR MEASURING WATER PRESENT IN VEGETATION AND OPERATION METHOD THEREOF**

VORRICHTUNG, LANDWIRTSCHAFTLICHE VORRICHTUNG UND VERFAHREN ZUR MESSUNG VON IN VEGETATION VORHANDENEM WASSER

DISPOSITIF, DISPOSITIF AGRICOLE ET PROCÉDÉ CORRESPONDANT POUR MESURER L'EAU PRÉSENTE DANS LA VÉGÉTATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.12.2018 PT 2018115239**
**28.03.2019 EP 19166018**

(43) Date of publication of application:
**10.11.2021 Bulletin 2021/45**

(73) Proprietor: **INESC TEC - Instituto de Engenharia de Sistemas e Computadores, Tecnologia e Ciência 4200-465 Porto (PT)**

(72) Inventors:
• **DUARTE DOS SANTOS, Manuel Cândido 4200-465 Porto (PT)**
• **BATISTA NEVES DOS SANTOS, Filipe 4200-465 Porto (PT)**
• **GOMES BORGES, Rodrigo 4200-465 Porto (PT)**

(74) Representative: **Patentree Edificio Net Rua de Salazares, 842 4149-002 Porto (PT)**

(56) References cited:
**ES-A1- 2 385 942**

• **FAWWAZ T ULABY ET AL: "Microwave Attenuation Properties of Vegetation Canopies", IEEE TRANSACTIONS ON GEOSCIENCE AND REMOTE SENSING, IEEE SERVICE CENTER, PISCATAWAY, NJ, US, vol. 44, no. 5, 1 September 1985 (1985-09-01), pages 746-753, XP011158947, ISSN: 0196-2892**
• **OGAWA Y ET AL: "Monitoring of water/ice state using millimeter waves for the agricultural field", INFRARED AND MILLIMETER WAVES, 2004 AND 12TH INTERNATIONAL CONFERENCE ON TERAHERTZ ELECTRONICS, 2004. CONFERENCE DIGEST OF THE 2004 JOINT 29 TH INTERNATIONAL CONFERENCE ON KARLSRUHE, GERMANY SEPT. 27 - OCT. 1, 2004, PISCATAWAY, NJ, USA,IEEE, 27 September 2004 (2004-09-27), pages 451-452, XP010795056, ISBN: 978-0-7803-8490-3**

- DOMINGO SANCHO-KNAPIK ET AL: "Microwave-band (1730MHz) accurately estimates the relative water content in poplar leaves. A comparison with a near infrared water index (/)", AGRICULTURAL AND FOREST METEOROLOGY, ELSEVIER, AMSTERDAM, NL, vol. 151, no. 7, 27 January 2011 (2011-01-27), pages 827-832, XP028384106, ISSN: 0168-1923, DOI: 10.1016/J.AGRFORMET.2011.01.016 [retrieved on 2011-02-03]
- QUEMADA C ET AL: "A Real-Time Non-Destructive Water Status Monitoring System at Terahertz Band", 2018 15TH EUROPEAN RADAR CONFERENCE (EURAD), EUROPEAN MICROWAVE ASSOCIATION, 26 September 2018 (2018-09-26), pages 465-468, XP033453464, DOI: 10.23919/EURAD.2018.8546613

**Description**

**TECHNICAL FIELD**

The present disclosure relates to a device and agricultural device for measuring water present in vegetation using radio frequency attenuation, respective agricultural vehicle and operation method.

**BACKGROUND**

Water plays a central role in farming processes and this input must be tightly controlled and corrective and precise actions must be taken to support healthy crops. Thus, precision agriculture together with agricultural robotics has been exploiting the use of variable rate technologies (VRT) for controlling the rate of agronomic inputs (e.g. water) based on contextual features such as plant status and location.

A traditional VRT system only uses information provided by agro data cloud systems to determine the amount of agronomic input required to be applied. However, these systems do not get real-time feedback about the real requirements of the plant needs (e.g. water) and the real amount of products applied.

Real-time and remote solutions (using satellites or aerial vehicles) disclosed in prior art comprise mostly radar scatterometers or optical-based systems.

Radar scatterometers namely Synthetic Aperture Radars (SAR), can monitor vegetation phenology, using an imaging-based system that transmits microwaves and receives the reflections of the backscattered signals. Although satellite SAR systems are specialized in providing average water content of wide planting regions they do not have resolution to measuring water content at a single plant or even at leave-by-leave-scale (i.e. precision agriculture applications). Moreover, satellite SAR systems are strongly affected by the interference of other elements during the scattering processes such as the soil moisture, the surface type, the terrain topology, the techniques used during the cultivation process and the atmospheric transmission characteristics [2].

Optical-based methods can be used for water sensing by analyzing the reflected electromagnetic signal by vegetation canopies. This reflectance is largely affected by the biological properties of the plant, and in the regions of 0.7 to 1.4 $\mu$m (near-infrared wavelengths) and 1.3 to 2.5 $\mu$m (middle-infrared wavelengths) of the electromagnetic spectrum, the reflectance is affected by the quantity of water in the leaves [1]. However, the accuracy of optical-based methods is negatively affected by atmospheric conditions. Moreover, water sensing using optical-based methods is limited to the contribution of the superficial layers of the plants because only reflected information is used.

Document KR101824652 relates to a device for calculating the moisture content of a plant. The device for calculating the weight and moisture content of a plant includes a cultivating bed on which a medium is mounted, wherein nutrient solution is supplied to enable the plant to be grown, a weight measuring portion, and a control portion. The weight measuring portion is configured to measure the weight of the medium mounted on the cultivating bed by floating the cultivating bed in the air. The control portion is configured to collect a measurement value from the weight measuring portion and calculate the weight and the water content of the plant by using the collected measurement value.

The prior art does not provide feasible practical solutions of measuring water content at a plant scale that can be adapted to different atmospheric and terrain conditions.

References

[1] E.R. Hunt and Barrett N Rock. Detection of changes in leaf water content using near- and middle-infrared reflectances. Remote Sensing of Environment, 30(1):43 - 54, 1989.

[2] G. Schiavon, D. Solimini, and A. Burini. Sensitivity of multi-temporal high resolution polarimetric C and L-band SAR to grapes in vineyards. In 2007 IEEE International Geoscience and Remote Sensing Symposium, pages 3651-3654, July 2007.

Document FAWWAZ T ULABY ET AL: "Microwave Attenuation Properties of Vegetation Canopies", IEEE TRANSACTIONS ON GEOSCIENCE AND REMOTE SENSING, IEEE SERVICE CENTER, PISCATAWAY, NJ, US, vol. 44, no. 5, 1 September 1985 (1985-09-01), pages 746-753, XP011158947, ISSN: 0196-2892, relates to a mobile agricultural measurement device (boom truck) carrying a microwave (radar) antenna, driving through an agricultural field and pulling a microwave receiver such that microwaves are transmitted through the plant material (live crops in a field, e.g. wheat or soy) at multiple locations in the whole field, taking into account stalks, leaves and fruits of the plants. The purpose is to gain insight into the microwave attenuation caused by plant material and specifically the attenuation's dependency on polarization, frequency and incident angle of the microwaves and on the plant part. Data is taken and electronically processed for different heights of plant material and along a row of plants, i.e. the data allows generating an 2D map image of the vegetation which shows the microwave attenuation. D1 does not mention the generation of such a 2D image, and does not suggest to use such microwave transmission/attenuation measurements to determine the actual plant water content.

Document OGAWA Y ET AL: "Monitoring of water/ice state using millimeter waves for the agricultural field", INFRARED AND MILLIMETER WAVES, 2004 AND 12TH INTERNATIONAL CONFERENCE ON TERAHERTZ ELECTRONICS, 2004. CONFERENCE DIGEST OF THE 2004 JOINT 29TH INTERNATIONAL CONFERENCE ON KARLSRUHE, GERMANY SEPT. 27 - OCT. 1, 2004, PISCATAWAY, NJ, USA, IEEE, 27 September 2004, (2004-09-27), pages 451-452, XP010795056, ISBN: 978-0-7803-8490-3, relates to measuring plant water present in vegetation for agricultural purposes by means of radiation in the microwave frequency range, and discloses a method and device to monitor the moisture of a living plant's leaves by measuring the transmittance of millimetre waves through them. This document states that this method and device can be used in agriculture for timing the watering of plants. The emitter/receiver device of this document is small and easily portable. This document has an electronic data processor connected and configured to receive the signal from the receiver, which can be used to produce a 2D representation of plant water content over time. This document, however, does not disclose that the electronic data processor also is connected and configured to control the beam displacement, or configured to generate spatial 2D images of the vegetation.

Document DOMINGO SANCHO-KNAPIK ET AL: "Microwave-band (1730MHz) accurately estimates the relative water content in poplar leaves. A comparison with a near infrared water index", AGRICULTURAL AND FOREST METEOROLOGY, ELSEVIER, AMSTERDAM, NL, vol. 151, no. 7, 27 January 2011 (2011-01-27), pages 827-832, XP028384106, ISSN:0168-1923, DOI: 10.1016/J.AGRFORMET.2011.01.016, relates to measuring plant water present in vegetation for agricultural purposes by means of radiation in the microwave frequency range, and describes water management for agricultural plants/crops, specifically to accurate irrigation control, and discloses a laboratory-scale initial study for developing a commercial apparatus enabling the determination of plant water status under field conditions using a microwave antenna. This document, does not disclose any details regarding the practical implementation, e.g. the (control of) direction of the beam or the movement of the device along plants in an actual agricultural field. Besides, the measurement principle of this document is based on the change of impedance of the antenna in response to the presence of water (e.g. in the form of plant water) in its vicinity and not actually an image-generating microwave attenuation measurement.

These facts are disclosed in order to illustrate the technical problem addressed by the present disclosure.

## GENERAL DESCRIPTION

The invention is defined by a device for measuring plant water present in vegetation as present in claim 1 and a method for measuring plant water present in vegetation as present in claim 15.

This document discloses a method and device for measuring the water content in plants using radio frequency attenuation for real-time water sensing. The method comprises according to an embodiment transmitting electromagnetic radiation with frequencies in particular above 20 GHz on one side of a plant, receive the transmitted signal on the other side of the said plant, and analyze the attenuation of the signal caused by the water content of the plant being irradiated. The implemented approach includes analyzing the attenuation caused by leaves, plants, and elements containing water over a transmission between a pair of high gain antennas.

The presented method and device are immune to dry elements that obstruct the view or to illuminations changes, which would impair the measurement by an optical based system.

In an embodiment the system can be used in combination with a spraying system, analysing the plants right before each spraying passage and right after the said passage, giving a real-time accurate feedback of the amount of product sprayed.

In an embodiment of an anti-drift panel/drift recovery-based sprayer the system can monitor the water quantity right before the product application and monitor water quantity application on leaf and plant surfaces, without need to be in contact with the plants (as the optical based systems require).

The antenna array system can be attached to any place of mobile machinery (autonomous or non-autonomous) and/or in machinery implemented in such way that the plants will be in the middle of antenna array system. To avoid the collision between antenna array system and the plants an active pan and tilt system may be used to control the antenna array system posture.

It is disclosed a device for measuring plant water present in vegetation comprising:

a microwave emitter of a displaceable beam;
a microwave receiver;
an electronic data processor connected to receive a signal from the microwave receiver; wherein the microwave emitter is arranged to direct said displaceable beam towards the microwave receiver for measuring attenuation of the beam passing through each of a plurality of locations of the vegetation;

wherein the electronic data processor is configured for calculating the attenuation of the displaceable beam between the emitter and receiver to obtain a 2D image corresponding to the measurement of water present in the vegetation, that is, to obtain a spatial 2D image of the vegetation wherein the image corresponds to the calculated attenuation and hence the measurement of water present in the vegetation.

Alternatively, by adequate displacing of the beam (or beams) a 3D image can be obtained. Also, by steering or displacing the beam at an inclined angle, in relation to the horizontal plane, towards the vegetation, it is then obtained information in respect of the water measurement, along an horizontal axis along a direction towards the vegetation, in order to obtain a 3D image or a 2.5D image (a 3D image where there is limited information along one or more of 3 orthogonal axis).

In an embodiment, the microwave receiver comprises a plurality of individual receivers.

In an embodiment, the microwave receiver comprises a plurality of individual microwave receivers linearly arranged and distributed in particular along a vertical direction.

In an embodiment, the microwave emitter is arranged to direct said displaceable beam towards each of said individual microwave receivers.

In an embodiment, the microwave emitter is arranged to direct said displaceable beam towards one by one of said individual microwave receivers.

In an embodiment, the microwave emitter comprises a steerable beam antenna, in particular a steerable beam phase-shift antenna, for directing said microwave beam between each of said individual microwave receivers.

In an embodiment, the microwave emitter comprises a plurality of individual emitters which are individually switchable for directing said microwave beam between each of said individual microwave receivers.

In an embodiment, the microwave emitter comprises a plurality of individual microwave emitter linearly arranged and distributed in particular along a vertical direction.

In an embodiment, said beam is displaceable by the device being movable along the vegetation to be measured in particular along a horizontal direction, in particular the microwave emitter and microwave receiver being jointly mounted in the device, further in particular the microwave emitter and microwave receiver being jointly mounted in opposite ends of an arched structure such that the vegetation can pass below the arch between the emitter and the receiver.

In an embodiment, the device is a vehicle, namely an agricultural vehicle, and the microwave emitter and microwave receiver are mounted on the vehicle for moving along the vegetation.

An embodiment comprises a satellite geolocation sensor, a radiofrequency geolocation sensor or a displacement sensor for synchronizing the measurement of water present in the vegetation with the device location.

An embodiment comprises a second microwave emitter and a second microwave receiver mounted on the vehicle for moving along the vegetation, such that the second emitter-receiver pair measure attenuation of the beam passing through each of a plurality of locations of the vegetation after the first emitter-receiver pair have measured attenuation of the beam passing through each of the same plurality of locations of the vegetation.

In an embodiment, the electronic data processor is configured for calculating the difference in attenuation of the displaceable beam between the first emitter-receiver pair and the second emitter-receiver pair to obtain a 2D image corresponding to the difference in the measurement of water present in the vegetation.

An embodiment comprises a dispenser for applying the treatment and being arranged to:

measure the water present in the vegetation before applying the treatment and adjust the amount of treatment applied in respect of the measured water; and/or

measure the water present in the vegetation before and after applying the treatment for calculating the difference in the measurement of water present in the vegetation to measure the applied treatment.

It is also disclosed a method of operation of a device, namely an agricultural device, for measuring plant water present in vegetation, said device comprising a microwave emitter of a displaceable beam; a microwave receiver; an electronic data processor connected to receive a signal from the microwave receiver; said method comprising:

directing said displaceable beam by the microwave emitter towards the microwave receiver for measuring attenuation of the beam passing through each of a plurality of locations of the vegetation;

calculating, by the electronic data processor, the attenuation of the displaceable beam between the emitter and receiver to obtain a 2D image corresponding to the measurement of water present in the vegetation.

**BRIEF DESCRIPTION OF THE DRAWINGS**

The following figures provide preferred embodiments for illustrating the disclosure and should not be seen as limiting the scope of invention.

**Figure 1:** Schematic representation of an overview of an embodiment of the disclosed device.

**Figure 2A and Figure 2B:** Schematic representations of views of possible embodiments using multiple emitting and receiving elements and their direct radiation pathways

**Figure 3:** Schematic representation of a diagram of an embodiment of the antenna array with switches.

**Figure 4:** Schematic representation of a view of an embodiment of antenna arrays with phase shifters.

**Figure 5:** Schematic representation of a view of an embodiment of the disclosed device combined with a vehicle and a spraying system for agriculture applications.

**Figure 6:** Comparison between the real image of the cactus-like plant and the resulting image after the analysis of the collected data obtained according to an embodiment of the disclosure.

**Figure 7:** Leaf with a dried region (bottom) and the respective result of the test scan obtained according to an embodiment of the disclosure.

**Figure 8A, Figure 8B, and Figure 8C:** Images of the analysis of a green leaf (A), the analysis of the same leaf but dry (B) and the difference between the two images (C) obtained according to an embodiment of the disclosure.

**Figure 9A, Figure 9B, and Figure 9C:** Images of a green leaf before (A) and after having been sprayed with water hence with water on its surface (B) and the difference between the two images (C) obtained according to an embodiment of the disclosure.

**DETAILED DESCRIPTION**

This document discloses a method and device for remote water sensing using transmitted microwaves, and more particularly for measuring water content in a plant. Unlike the optical-based methods that measure and analyse the visible and infrared signals reflected by the object of interest, the method and device of this document uses transmitted microwaves enabling sensing deeper plant layers and being immune to the interference caused by illumination changes or by the presence of dry elements on the accuracy of the predicted water content values.

The water content measurement can be used to characterize the water status of plants or as an input to the control other devices and systems (e.g. spraying system), adjusting automatically the amount of the agronomic inputs to be sprayed. The disclosed device and method is capable to create a 2D or 2.5D, or even three-dimensional, map of water content present in the plant or group of plants.

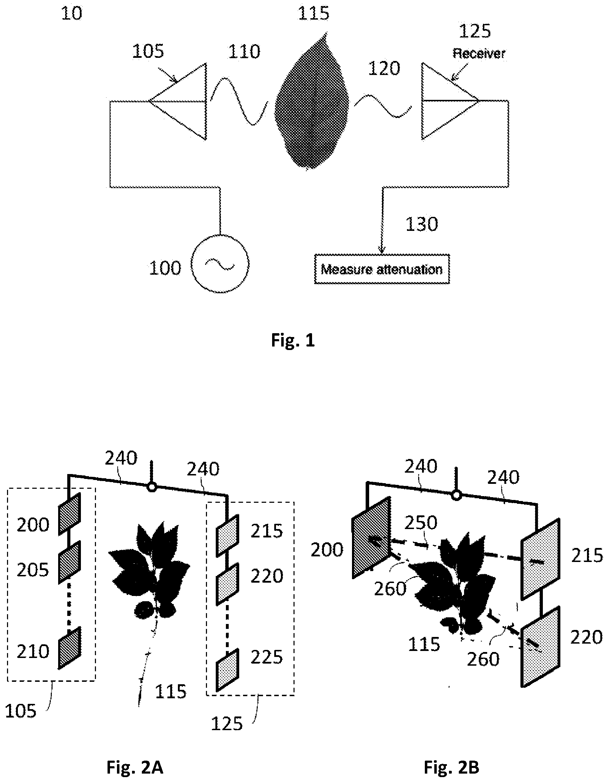

Reference is made to **Fig. 1** which is a schematic view of the device and illustrates the method to measure water content in plants. As depicted by **Fig. 1,** the device **10** comprises an emitter **105** that generates an electromagnetic radiation **110** (e.g. microwaves) towards a plant or a region of the plant (e.g. leaves) **115.**

The generated electromagnetic radiation **110** may be, but is not limited to, frequencies around 20 GHz, due to the effects of water presence in the signal radiation path, which is established between an emitter **105** and a receiver **125.** In fact, the receiver measures a peak of attenuation around 20 GHz when water (gas or liquid form) or a water containing object is between the receiver and the emitter antennas (direct radiation path). The said electromagnetic radiation **110** is transmitted but attenuated along the plant **115.** The transmitted electromagnetic radiation **120** is sensed by the receiver **125** and the signal attenuation is measured by the unit **130.** The attenuation caused by the plant **115** leads to a loss in power of the said generated electromagnetic radiation **110,** depending on the water content of the plant **115.**

In free space conditions meaning absence of any object or plant 115 in the direct radiation path between the emitter and the receiver, the attenuation in the radiated signal is given by free space path loss ($L_{fs}$) calculated by the **Equation 1,** where $f$ is the frequency, $c$ is the speed of light in the vacuum and D is the distance between the emitter and the receiver.

$$L_{fs} = 20\log_{10}\left(\frac{4\pi fD}{c}\right) \text{ (dB)} \quad \textbf{(Equation 1)}$$

In presence of a water-containing object (e.g. **plant**) the measured signal loss between the emitter and the receiver is significantly increased when compared to the signal loss value obtained in the calibration, which can be performed in free space condition ($L_{fs}$) or in a real environment (humid air) without a plant in the direct radiation path.

The plant water content is predicted by the differential amount of signal loss (attenuation) between the plant measure and the calibration.

Frequencies in the range of 20GHz were applied in this example because they can go through dried plants without suffering signal loss so not changing $L_{fs}$ value.

Higher frequencies than 20 GHz may be applied to improve water sensing sensitivity. However, higher frequencies lead to increased free space power loss what requires decreasing the distance between the emitter **105** and the receiver **125** for the same radiated power. Improved spatial resolution may be achieved by the use of higher frequencies than 20GHz. Other different frequencies may be selected and used to avoid excessive losses due to specific use cases or to avoid reflections occurring at a specific frequency. Also, different frequencies may be used simultaneously, in order to electronically or computationally select the frequency signal or signals providing the best measurement.

In an alternative embodiment power reflection may be also used to predict the plant water content. However, the power reflected by the said plant is extremely low which leads to decreased sensitivity comparing to the disclosed method using attenuation between the emitter and the receiver.

In order to water sensing different plants with different shapes and sizes, a variable number of emitters and receivers can be used as disclosed in **Fig.2A.** In this embodiment, the emitter element **105** may be an array comprising at least an emitter antenna **200** and the receiver element **125** may be an array with at least a receiver antenna **215**. The term emitter/receiver pair will be used to describe any combination of an emitter from a multitude of emitters in an array and a receiver from a multitude of receivers in an array that can establish a link.

The said emitter array **105** and the said receiver array **125** are connected by a structure **240** (e.g. metallic bar) to control the distance between them, i.e. the direct radiation path length. The said structure **240** may be connected to a mobile structure (e.g. robotic arm) that can move the said emitter and receiver arrays to measure the water content in different points of the plant **115.**

Reference is made to **Fig. 2B** to explain the different radiation paths that characterize each emitter/receiver pair. For example, the radiation path **250** characterize the link between the emitter **200** and the receiver **215** while the radiation path **260** characterize the link between the emitter **200** and the receiver **220.** Therefore, the same emitter can provide different radiation paths which can be sensed by different receivers. This increase in the number of direct radiation paths can be used to improve the spatial resolution and the accuracy of the measurement because each direct radiation path between an emitter/receiver pair can be used to measure an attenuation value, and thus water content, that can be mapped. This map can be presented as a water content image.

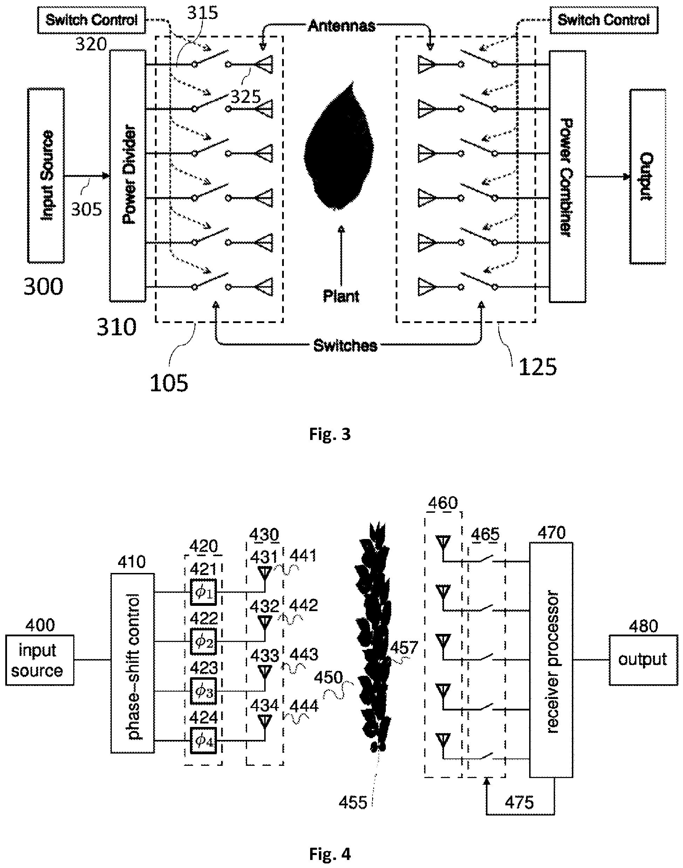

To mitigate the interference caused by reflections on the water sensing, this disclosure implements isolation between signals paths by controlling along time the operation of non-simultaneous emitter/receiver pairs. Reference is made to **Fig. 3** to explain how the emitter/receiver pairs can be established.

References is made to **Fig. 3** to disclose the electric-scan based in switch controlling. An input source **300** generates the input signal **305** which is injected in a power divider **310**. This power divider **310** is composed of as many power channels **315** as the number of emitting antennas. Each power channel **315** is connected to a switch control **320** that controls the power delivered to each emitting antenna **325.**

Each emitter/receiver pair is activated at each given moment, with the intensity of the emitted signal being attenuated by the objects and plants in the signal path between the emitter/receiver pair. In this embodiment, the electrical scan consists of controlling the switches connected to each antenna as shown in Fig. 3 turning on and off the different emitter/receiver pairs in the arrays to scan the whole area.

Reference is made to **Fig. 4** to describe a preferable embodiment of the disclosure disclosed herein comprising electric-scan based in phase shifting for beam steering control. An input source **400** generates the input signal which is injected in a phase-shift control **410**. This phase-shift control **410** is composed of as many power channels as the number of emitting antennas. Each power channel supplies a phase shifter **421-424**. A phase control module **410** individually controls each phase shifter **421-424**. Each phase shifter **421-424** is connected to an emitting antenna **431-434** that emits an emitted signal **441-444**. At least two phase shifters **421** and **422** and correspondent two emitting antennas **431** and **432** are required for steering the emitted signals **441-442** in to an emitted beam **450.**

This electronically steerable array of antennas enables the control of the direction of the emitted beam **450** (beam). In this configuration, all the emitting antennas of the array **450** emit at the same time. The combination of the individual emitted signals of each antenna of the array creates an emitted beam, directing the radiation pattern of the array in a specific direction.

The emitted beam angle control is achieved by shifting the phase of the signal emitted from each emitting antenna to provide constructive or destructive interference so as to steer the emitted beam **450** in the desired direction. The direction of the emitted beam **450** is therefore changed by controlling the phase shifters **420** connected to each radiating elements of the array **430**.

The beam steering of the array disclosed in the **Fig. 4** is performed by controlling the phase of the signal for each antenna. The direction of the beam is defined by the phase difference between each consecutive element of the array. This phase difference ($\Delta\varphi$) is obtained using the **Equation 2,** where d is the predetermined distance between the radiating elements, $\lambda$ is the wavelength (e.g. 20GHz) and $\theta_s$ is the desired angle of the beam.

$$\Delta\varphi = \frac{360^o \times d \times sin(\theta_s)}{\lambda} \qquad \textbf{(Equation 2)}$$

The emitted beam **450** suffers attenuation while it is transmitted along the plant **455,** continuing as transmitted beam **457** towards the receiving elements **460**. Each receiving element is connected to a switch **465,** which is controlled by switch control **475,** turning on and off individual switches from **465**. The receiver processor **470** determines the water content value, based in measured the attenuation values, to each element of the map. At each time the receiver processor **470** has access to the present emitted beam angle which can be used to weight or correct the map of attenuations and consequent water content values that are outputted **480**.

The determined attenuation (power loss) values are normalized to the range of 0 to 1 using the maximum and minimum values of the matrix with the absolute values. A grayscale image of the object, plant or leaf is therefore generated where the brightest part of the image represents the area with the greatest attenuation and, therefore, a greater water quantity, whereas, the darkest areas of the image represents a lower signal attenuation corresponding to areas with less water content.

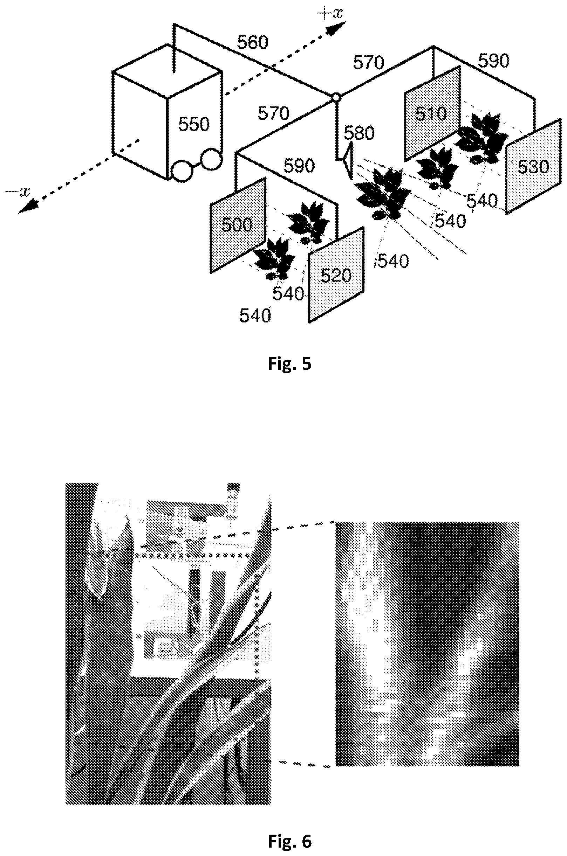

Reference is made to **Fig. 5** to disclose a preferential embodiment of the present disclosure for agriculture applications. In this embodiment a vehicle **550** is connected through an element **560** to an apparatus to automatically spray **580** an agronomical product. In this embodiment, and assuming that the said vehicle **550** is moving along the positive x axis, the plant **540** water content is determined by the device that comprises the emitter/receiver pair **510** and **530,** connected to a fixed structure **590** to ensure a constant distance, prior to the spray step. A second emitter/receiver pair **500** and **520** is used to provide a second water content measurement that is carried out after the spraying step. The elements **500** and **520** are arranged in face-to-face manner where the plant **540** is between them. The emitter/receiver pair are attached to the sprayer element **580** through an element **570**. The plant water content value determined by the pair **510** and **530** can be used to control the amount of agronomical product sprayed by the element **580**. The vegetation elements **540** are sprayed using a sprayer **580** that is attached to a vehicle **550** that moves in parallel with the alignment of the vegetation (x axis).

Based on the differences of attenuation (water content) sensed by the two said emitter/receiver pairs **(510, 530 and 500, 520)** the amount of sprayed agronomical product is determined as well as its position and scattering on the plant considering the data collected during the scanning step (beam steering). In case of low product distribution, the vehicle **550** moves backwards, repeating the spraying step. Each transmitter transmits a signal with transmit power and known frequencies. The positioning of the vehicle is known over time. Along the +/- x offset each receiver that records the attenuation over time. According to the direction of travel of the vehicle **550,** one receiver will record the power of the received signal before spraying and the other the power of the signal after spraying. The difference in power attenuation received, after correction of distance between receivers, allows to determine the quality of spraying.

The longitudinal velocity of the vehicle can be changed in order to increase or decrease the longitudinal map detail as data collection is synchronised with the displacement of the vehicle, for example by satellite or radiofrequency geolocation, or for example by a rotational encoder placed at the vehicle wheels or transmission.

The signal attenuation that has a direct relation to the water quantity is integrated with the system geolocation to build a three-dimensional map where each observed spot, defined in a North-East-Up referential coordinate system. This three-dimensional signal attenuation map is after converted to a three-dimensional water content map.

In an alternative embodiment, the present disclosure can be used to continuous monitoring of plant water content as it is possible to repeat previous scans to obtain data over time.

Typically, measurements can be obtained at multiple times per second, which enables sampling rates of for example 1000 measurements per metre of vehicle displacement, such that the horizontal (in the direction of displacement) sampling points per metre can be larger or even much larger than the vertical (along the distribution of antenna emitter/receiver pairs) sampling points per metre. As a consequence, the horizontal image resolution can be larger or much larger than the vertical image resolution. Advantageously, this data can be used by computer methods to produce more detailed data on plant growth.

In an embodiment, fruit content can also be measured as fruits contain water. In a further embodiment, fruit water content can be also discriminated from leaf water content as fruits will cause much larger signal attenuation. In another further embodiment, fruit water content can be also discriminated from leaf water content as a vehicle applies a phytosanitary treatment by taking two consecutive measurements and using the fact that leaves will cause differential measurements reflecting the water content of the treatment applied whilst fruit will cause the signal to reach a maximum signal threshold and thus no differential measurement.

The following pertains to experimental results. The laboratorial tests carried out to evaluate the method and device disclosed herein used two high gain antennas mounted on a claw. This claw was placed on the end of a robotic arm, which allowed for the scanning of the object being measured. The pair of high gain antennas was connected to a vector network analyzer (VNA) by coaxial cables to measure the S-parameters of the transmitted signal, more precisely, the $S_{12}$ parameter. The model of the VNA was the E8363B PNA Series Network Analyzer (10 MHz to 40 GHz) from Agilent Technologies and the model of the coaxial cables were the KMM24 from Thorlabs and the KBL-2FT-PHS+ from Minicircuits, both prepared for the high frequencies in use during the tests.

For each test, a test element was placed between the emitter/receiver pair to be scanned. The area of this scanning process was defined in the Matlab script with the desired step size. The maximum value of this step size was 3 mm, dictated by the maximum precision of the robotic arm. The maximum size of the scanning area was a 20 cm by 20 cm square, due to physical limitations of the robotic arm. The test sequence consisted of acquiring the $S_{12}$ parameter measured by the VNA on the initial point, send a request to move the robotic arm to the next position, acquire the $S_{12}$ parameter of that position and so on until all the positions defined by the pre-determined area for the scanning process were achieved.

Water content measurements were carried out in different objects, plants, and leaves. The size of the objects and plants was limited by the distance between the emitter/receiver pair. This distance was dictated by the size of the two claws used. With the first claw, the distance between antennas was 20 cm, whereas, with the second claw, this distance was 9 cm. Initially, the tests were performed using the first claw, although the distance between the antennas meant that sometimes the VNA measured really low values, in the range of -70 dB to -80 dB for the $S_{12}$ parameter. This value was at the end of the VNA measuring range, and for that reason, the second claw was created to improve the tests performance and to increase the range of intensity values acquired by the VNA.

The different tests performed with plants and leaves will be presented in the following paragraphs. The leaves used during the tests were suspended from a wooden structure so that the only object in the middle of the emitter/receiver pair was the leaves. The same type of leaf was used throughout the tests to decrease the number of variables. As for the plant, its size was small enough to be fitted between the antennas and to allow for the scanning procedure.

A test using cactus-like plant was performed, as presented in **Fig. 6.** This plant was chosen due to its high content in water, causing a greater attenuation on the intensity of the signal. The analysis of the collected data for this test confirms that the signal is strongly attenuated when the signal is passing through the plant. This is perceptible by analyzing the images obtained from the collected data, where the brightest parts of the images correspond to areas where a leaf of the plant is directly in the middle of the emitter/receiver pair, and the darkest areas correspond to the void spaces between each leaf, as observed in **Fig. 6.** The scanning area of this test was a rectangle of 20 cm by 15 cm with a step size of 5 mm, corresponding to 1200 different points. The results are from three different frequencies (19.5 GHz, 19.75 GHz and 20 GHz) as well as the average of the attenuation in the range of 19.5 to 20 GHz. The analysis of the resulting images reveals better results using the average of the frequency range.

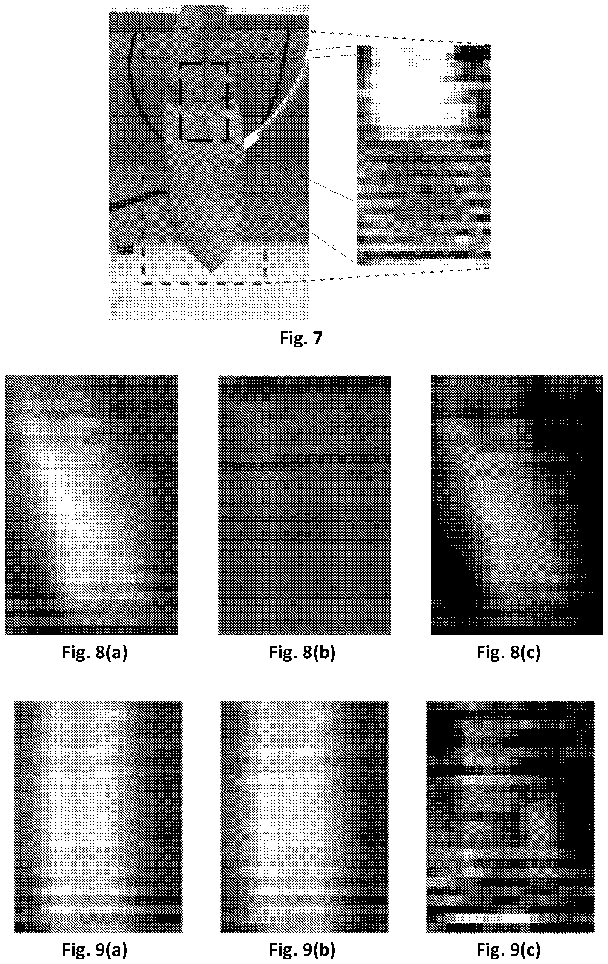

The method and device disclosed herein is able to scan and measure in detail the water content of object of interest with regions with different hydration levels. The disclosure was tested using a leaf with a green region (upper, leaf petiole) and a dry region (bottom, leaf apex) as illustrated on the left side of **Fig. 7.** The leaf was directly extracted from the tree in this condition, with almost half of the leaf in a dry state. The scanning area of this test was a rectangle of 12 cm by 7 cm with a step size of 0.4 cm, corresponding to 540 different points. As observed in **Fig. 7,** the disclosed invention measured higher water content and further presented brighter pixels in the upper region of the generated 2D image, which corresponds to the green region observed near to the leaf petiole. Conversely, the middle and bottom part of the generated image comprised darker pixels, which resulted from the reduced signal attenuation suffered by the microwaves transmitted along this dry region of the leaf near the apex that was measured by the disclosure. This correlates to the real image since the brown portion of the leaf has less water content in comparison to the green part.

The disclosure is also able to measure water content along time. This test consisted of starting with a green leaf freshly taken from a tree, measure its mass using a high precision scale and perform a scanning test. Next, the leaf was left to dry naturally for two days, before being again weighted and scanned. For this test, the size of the scanning area was a 12 cm by 8 cm rectangle with a step size of 0.4 cm, corresponding to a total of 600 different points.

The weight of the leaf decreased between each scanning procedure, starting at 1.1094 grams and finishing at 0.3908 grams. This loss of weight by the leaf is mainly due to the evaporation of water during the natural drying process, since the physical structure of the leaf stayed intact during the drying process and the scanning procedures. This loss of weight corresponds to a loss of water by the leaf, thus affecting each test scan results. The results of this analysis are shown in **Fig. 8,** where **Fig. 8a** represents the result of the scan of the green leaf (with a mass of 1.1094 grams), **Fig. 8b** represents the result of the dry leaf (with a mass of 0.3908 grams) and image **Fig. 8c** is the difference between the results of the green leaf and the dry leaf. The images were obtained by normalizing both matrices with the global maximum and minimum between the matrices acquired from both scanning procedures. The fact that the green leaf result has a brighter area in the zone of the leaf, whereas, on the dry leaf the result is an image almost black and uniform, indicates a clear sensibility of the system to the water content inside the leaf. The lack of a clear contrast between the dry leaf and the area of the image without the leaf in **Fig. 8b** indicates that there is not a significant attenuation of the signal due to the lack of water on the dry leaf.

The analysis of the sum of all the elements of the normalized matrices also gives a clear indication of the effects of the water content of the leaf on the signals' attenuation. The sum of the values of the normalized matrix of the green leaf was 262.20, whereas, for the dry leaf this sum was 156.47. This difference is explained due to the fact that a greater attenuation of the signal is represented by a number closer to 1 on the matrix, thus giving a bigger sum of the elements of the matrix of the green leaf.

The analysis of the difference between the maximum and minimum values recorded by the VNA also reveals some information about the leaves water content. For the data recorded for the green leaf, the difference between the maximum and the minimum values was 6.59 dB, whereas, on the dry leaf, this value was 2.57 dB. This difference of 4.02 dB between these two values reveals a clear indication of a greater effect on the signals' attenuation with the green leaf.

The same test was repeated using a smaller distance between antennas, this time with the scanning procedure being repeated six times over the course of two days and the mass of the leaf being measured before each scan. The values of the leaves mass for each scanning procedure are shown in **Table 1,** with the first three scans being performed on the first day and the following scans on the second day.

**Table 1** - Leaves mass at each scan (test) and corresponding difference between the maximum and minimum values of the matrices of each test result.

|  | Day 1 | | | Day 2 | | |
| --- | --- | --- | --- | --- | --- | --- |
|  | **Test 1** | **Test 2** | **Test 3** | **Test 4** | **Test 5** | **Test 6** |
| **Mass (g)** | 1.0472 | 0.9741 | 0.9336 | 0.6181 | 0.5652 | 0.5311 |
| **Difference Max - Min (dB)** | 3.87 | 3.74 | 3.73 | 1.92 | 1.86 | 1.58 |

The analysis of the difference between the maximum and minimum values of the data collected for each test reveals an indication of the effect of the leaves water content on the signal attenuation. The higher the value of the leaves mass (corresponding to a higher water content), the higher the attenuation on the signals' intensity, represented by a greater difference between the maximum and minimum values of the of the tests performed on the first day in relation to the tests on the second day, as shown in **Table 1.**

Reference is made to **Fig. 9** to disclose the results on water sensing after applying a water-based agricultural treatment. The purpose of this test was to check the sensitivity of the disclosure to small changes in the quantity and position of water on a green leaf. The scanning area of this test was a 10 cm by 7 cm rectangle with a step size of 0.4 cm, corresponding to 450 different points. Water content was measured at two different timepoints - the first immediately after water spraying (Fig. 9a) and the second, two hours and half later (Fig. 9b). Despite the small visual difference between **Fig. 9a** and **Fig. 9b,** the resulting difference of these two images shows some bright pixels in the leaf apex that is shown in the lower part of the image **(Fig. 9c).** These pixels correspond to a leaf region where the signal attenuation due to the presence of water in the leaf is higher. These bright pixels are located in the leaf apex (lower part of **Fig. 9c)** as expected since gravity pulls down the drops of water towards the apex. The fact that the test took about two and a half hours to be completed was also a factor to this result as some of the water on the top part of the leaf evaporated before the scan passed over that part of the leaf. The analysis of the pixel values of each image for the two timepoints also revealed that there is a bigger sum for the matrix of the leaf with water on its surface, which again confirms the effect of water on the signals' attenuation. The sum of the values of this matrix was 242.453, whereas, for the matrix of the values for the leaf without water, this sum corresponds to 230.723.

Preferably, for avoiding parallax measurement errors, the beam is unfocused, propagating in constant width, i.e. constant spatial beam profile - from each emitter, through the respective vegetation location to be measured, to the respective receiver (i.e. without concentration or spreading of the beam).

When using a vehicle, the horizontal resolution may be variable, for example taking into account the precision of the geolocation or the displacement sensor. Thus, the horizontal and vertical resolution may differ when using a vehicle.

The term "comprising" whenever used in this document is intended to indicate the presence of stated features, integers, steps, components, but not to preclude the presence or addition of one or more other features, integers, steps, components or groups thereof.

It is to be appreciated that certain embodiments of the disclosure as described herein may be incorporated as code (e.g., a software algorithm or program) residing in firmware and/or on computer useable medium having control logic for enabling execution on a computer system having a computer processor, such as any of the servers described herein. Such a computer system typically includes memory storage configured to provide output from execution of the code which configures a processor in accordance with the execution. The code can be arranged as firmware or software to configure the machine in which it is executed to perform the associated functions, as described herein.

The disclosure should not be seen in any way restricted to the embodiments described and a person with ordinary skill in the art will foresee many possibilities to modifications thereof. The above described embodiments are combinable. The following claims further set out particular embodiments of the disclosure.

**Claims**

1.  Device for measuring plant (115, 455, 450) water present in vegetation comprising:

    a microwave emitter (105, 200, 500) of a beam, arranged such that the beam is displaceable to pass through each of a plurality of locations of the vegetation;
    a microwave receiver (125, 215, 220, 520);
    an electronic data processor connected to said microwave emitter and to said microwave receiver and configured to receive a signal from the microwave receiver (125, 215, 220, 520) and to control the displacement of said beam;
    wherein the microwave emitter (105, 200, 500) is arranged to direct said displaceable beam towards the microwave receiver for measuring attenuation of the beam passing through each of said plurality of locations of the vegetation;
    wherein the electronic data processor is configured for calculating the attenuation of the displaceable beam between the emitter and receiver to obtain a spatial 2D image of the vegetation wherein the image corresponds to the measurement of water present in the vegetation.

2.  Device for measuring plant (115, 455, 450) water present in vegetation according to the previous claim wherein the microwave receiver (125, 215, 220, 520) comprises a plurality of individual receivers.

3.  Device for measuring plant (115, 455, 450) water present in vegetation according to the previous claim wherein the microwave receiver comprises a plurality of individual microwave receivers (125, 215, 220, 520) linearly arranged and distributed, such that each receiver receives the beam passing through each of said plurality of locations of the vegetation, in particular the plurality of individual microwave receivers being linearly arranged and distributed along a vertical direction.

4.  Device for measuring plant (115, 455, 450) water present in vegetation according to any of the claims 2-3 wherein the microwave emitter (105, 200, 500) is arranged to direct said displaceable beam towards each of said individual microwave receivers.

5.  Device for measuring plant (115, 455, 450) water present in vegetation according to the previous claim wherein the microwave emitter (105, 200, 500) is arranged to direct said displaceable beam towards each of said individual microwave receivers (125, 215, 220, 520) one by one, such that each receiver receives the beam passing through each of said plurality of locations of the vegetation.

6.  Device for measuring plant (115, 455, 450) water present in vegetation according to any of the claims 2-5 wherein the microwave emitter comprises a steerable beam antenna, in particular a steerable beam phase-shift antenna, for directing said microwave beam between each of said individual microwave receivers (125, 215, 220, 520).

7.  Device for measuring plant (115, 455, 450) water present in vegetation according to any of the claims 2-6 wherein the microwave emitter (105, 200, 500) comprises a plurality of individual emitters which are individually switchable for directing said microwave beam between each of said individual microwave receivers (125, 215, 220, 520).

8.  Device for measuring plant (115, 455, 450) water present in vegetation according to the previous claim wherein the microwave emitter (105, 200, 500) comprises a plurality of individual microwave emitters (105, 200, 500) linearly arranged and distributed such that each receiver receives the beam emitted from each of said emitters passing through each of said plurality of locations of the vegetation, in particular the plurality of individual microwave emitters being linearly arranged and distributed along a vertical direction.

9. Device for measuring plant (115, 455, 450) water present in vegetation according to any of the previous claims wherein said beam is displaceable by the device being movable along the vegetation to be measured in particular along a horizontal direction, the microwave emitter and microwave receiver being jointly mounted in the device, further in particular the microwave emitter (105, 200, 500) and microwave receiver (125, 215, 220, 520) being jointly mounted in opposite ends of an arched structure.

10. Agricultural vehicle (550) comprising the device for measuring plant (115, 455, 450) water present in vegetation according to the previous claim wherein the microwave emitter (105, 200, 500) and microwave receiver (125, 215, 220, 520) are mounted on the vehicle (550) for moving along the vegetation such that the beam is displaceable to pass through each of the plurality of locations of the vegetation as the vehicle moves.

11. Agricultural vehicle (550) according to the previous claim comprising a satellite geolocation sensor, a radiofrequency geolocation sensor or a displacement sensor for synchronizing the measurement of water present in the vegetation with the device location determining which the plurality of locations of the vegetation is being measured as the vehicle (550) moves.

12. Agricultural vehicle (550) according to the previous claim comprising a second microwave emitter and a second microwave receiver mounted on the vehicle (550) for moving along the vegetation,
such that the second emitter-receiver pair measure attenuation of the beam passing through each of a plurality of locations of the vegetation after the first emitter-receiver pair have measured attenuation of the beam passing through each of the same plurality of locations of the vegetation.

13. Agricultural vehicle (550) according to the previous claim, wherein the electronic data processor is configured for calculating the difference in attenuation of the displaceable beam between the first emitter-receiver pair and the second emitter-receiver pair to obtain a 2D image corresponding to the difference in the measurement of water present in the vegetation.

14. Agricultural vehicle (550) according to the previous claim wherein the device for applying a water-based agricultural treatment to vegetation comprises a dispenser for applying the treatment and is arranged to:

measure the water present in the vegetation before applying the treatment and adjust the amount of treatment applied in respect of the measured water; and/or
measure the water present in the vegetation before and after applying the treatment for calculating the difference in the measurement of water present in the vegetation to measure the applied treatment.

15. Method of operation of an agricultural device for measuring plant (115, 455, 450) water present in vegetation, said device comprising a microwave emitter of a beam arranged such that the beam is displaceable to pass through each of a plurality of locations of the vegetation; a microwave receiver; an electronic data processor connected to said microwave emitter and to said microwave receiver and configured to receive a signal from the microwave receiver (125, 215, 220, 520) and to control the displacement of said beam; said method comprising:

moving said displaceable beam by the microwave emitter (105, 200, 500) towards the microwave receiver for measuring attenuation of the beam passing through each of a plurality of locations of the vegetation;
calculating, by the electronic data processor, the attenuation of the displaceable beam between the emitter and receiver to obtain a spatial 2D image of the vegetation wherein the image corresponds to the measurement of water present in the vegetation.

**Patentansprüche**

1. Vorrichtung zur Messung von in der Vegetation vorhandenem Pflanzenwasser (115, 455, 450), umfassend:

einen Mikrowellensender (105, 200, 500) mit einem Strahl, der so angeordnet ist, dass der Strahl verschiebbar ist, um durch jede einer Vielzahl von verschiedenen Stellen der Vegetation zu verlaufen;

einen Mikrowellenempfänger (125, 215, 220, 520);
einen elektronischen Datenprozessor, der mit dem genannten Mikrowellensender und dem genannten Mikrowellenempfänger verbunden und so konfiguriert ist, dass er ein Signal von dem Mikrowellenempfänger (125, 215, 220, 520) empfängt und die Verschiebung des genannten Strahls steuert;
wobei der Mikrowellensender (105, 200, 500) so angeordnet ist, dass er den genannten verschiebbaren Strahl auf den Mikrowellenempfänger richtet, um die Abschwächung des Strahls zu messen, der durch jede der genannten Vielzahl von Stellen der Vegetation verläuft;
wobei der elektronische Datenprozessor so konfiguriert ist, dass er die Abschwächung des verschiebbaren Strahls zwischen dem Sender und dem Empfänger berechnet, um ein räumliches 2D-Bild der Vegetation zu erhalten, wobei das Bild der Messung des in der Vegetation vorhandenen Wassers entspricht.

2. Vorrichtung zur Messung von in der Vegetation vorhandenem Pflanzenwasser (115, 455, 450) nach dem vorangehenden Anspruch, wobei der Mikrowellenempfänger (125, 215, 220, 520) eine Vielzahl von Einzelempfängern umfasst.

3. Vorrichtung zur Messung von in der Vegetation vorhandenem Pflanzenwasser (115, 455, 450) nach dem vorangehenden Anspruch, wobei der Mikrowellenempfänger eine Vielzahl von einzelnen Mikrowellenempfängern (125, 215, 220, 520) umfasst, die linear angeordnet und verteilt sind, so dass jeder Empfänger den Strahl empfängt, der durch jede der genannten Vielzahl von Stellen der Vegetation hindurchgeht, wobei die Vielzahl von einzelnen Mikrowellenempfängern insbesondere entlang einer vertikalen Richtung linear angeordnet und verteilt ist.

4. Vorrichtung zur Messung von in der Vegetation vorhandenem Pflanzenwasser (115, 455, 450) nach einem der Ansprüche 2-3, wobei der Mikrowellensender (105, 200, 500) so angeordnet ist, dass er den genannten verschiebbaren Strahl auf jeden der einzelnen Mikrowellenempfänger richtet.

5. Vorrichtung zur Messung von in der Vegetation vorhandenem Pflanzenwasser (115, 455, 450) nach dem vorangehenden Anspruch, wobei der Mikrowellensender (105, 200, 500) so angeordnet ist, dass er den genannten verschiebbaren Strahl nacheinander auf jeden der genannten einzelnen Mikrowellenempfänger (125, 215, 220, 520) richtet, so dass jeder Empfänger den Strahl empfängt, der durch jede der genannten Vielzahl von Stellen der Vegetation verläuft.

6. Vorrichtung zur Messung von in der Vegetation vorhandenem Pflanzenwasser (115, 455, 450) nach einem der Ansprüche 2-5, wobei der Mikrowellensender eine lenkbare Richtantenne, insbesondere eine steuerbare phasenverschiebare Richtantenne umfasst, um den genannten Mikrowellenstrahl zwischen jedem der genannten einzelnen Mikrowellenempfänger (125, 215, 220, 520) zu steuern.

7. Vorrichtung zur Messung von in der Vegetation vorhandenem Pflanzenwasser (115, 455, 450) nach einem der Ansprüche 2-6, wobei der Mikrowellensender (105, 200, 500) eine Vielzahl von einzelnen Sender umfasst, die einzeln schaltbar sind, um den genannten Mikrowellenstrahl zwischen jedem der genannten einzelnen Mikrowellenempfänger (125, 215, 220, 520) zu steuern.

8. Vorrichtung zur Messung von in der Vegetation vorhandenem Pflanzenwasser (115, 455, 450) nach dem vorangehenden Anspruch, wobei der Mikrowellensender (105, 200, 500) eine Vielzahl von einzelnen Mikrowellensender (105, 200, 500) umfasst, die linear angeordnet und verteilt sind, so dass jeder Empfänger den von jedem der genannten Sender ausgesandten Strahl empfängt, der durch jede der genannten Vielzahl von Stellen der Vegetation verläuft, wobei die Vielzahl von einzelnen Mikrowellensender insbesondere entlang einer vertikalen Richtung linear angeordnet und verteilt ist.

9. Vorrichtung zur Messung von in der Vegetation vorhandenem Pflanzenwasser (115, 455, 450) nach einem der vorangehenden Ansprüche, wobei der genannte Strahl dadurch verschiebbar ist, dass die Vorrichtung entlang der zu messenden Vegetation, insbesondere entlang einer horizontalen Richtung bewegbar ist, wobei der Mikrowellensender und der Mikrowellenempfänger gemeinsam in der Vorrichtung angebracht sind, wobei der Mikrowellensender (105, 200, 500) und der Mikrowellenempfänger (125, 215, 220, 520) ferner insbesondere gemeinsam an gegenüberliegenden Enden einer bogenförmigen Struktur angebracht sind.

10. Landwirtschaftliches Fahrzeug (550), umfassend die Vorrichtung zur Messung von in der Vegetation vorhandenem Pflanzenwasser (115, 455, 450) nach dem vorangehenden Anspruch, wobei der Mikrowellensender (105, 200, 500) und der Mikrowellenempfänger (125, 215, 220, 520) an dem Fahrzeug (550) angebracht sind, um sich entlang der Vegetation zu bewegen, so dass der Strahl verschiebbar ist, um durch jede der Vielzahl von Stellen der Vegetation zu verlaufen, während sich das Fahrzeug bewegt.

11. Landwirtschaftliches Fahrzeug (550) nach dem vorangehenden Anspruch, umfassend einen Sensor zur satellitengestützten Geolokalisierung, einen Sensor zur frequenzgestützten Geolokalisierung oder einen Wegsensor, um die Messung des in der Vegetation vorhandenen Wassers mit dem Standort der Vorrichtung zu synchronisieren und um zu bestimmen, welche der Vielzahl von Stellen der Vegetation gemessen wird, während sich das Fahrzeug (550) bewegt.

12. Landwirtschaftliches Fahrzeug (550) nach dem vorangehenden Anspruch, umfassend einen zweiten Mikrowellensender und einen zweiten Mikrowellenempfänger, die an dem Fahrzeug (550) angebracht sind, um sich entlang der Vegetation zu bewegen,
so dass das zweite Sender-Empfänger-Paar die Abschwächung des Strahls misst, der durch jede einer Vielzahl von Stellen der Vegetation verläuft, nachdem das erste Sender-Empfänger-Paar die Abschwächung des Strahls gemessen hat, der durch jede der gleichen Vielzahl von Stellen der Vegetation verläuft.

13. Landwirtschaftliches Fahrzeug (550) nach dem vorangehenden Anspruch, wobei der elektronische Datenprozessor so konfiguriert ist, dass er den Unterschied in der Abschwächung des verschiebbaren Strahls zwischen dem ersten Sender-Empfänger-Paar und dem zweiten Sender-Empfänger-Paar berechnet, um ein 2D-Bild zu erhalten, das dem Unterschied in der Messung des in der Vegetation vorhandenen Wassers entspricht.

14. Landwirtschaftliches Fahrzeug (550) nach dem vorangehenden Anspruch, wobei die Vorrichtung zum Ausbringen einer landwirtschaftlichen Behandlung auf Wasserbasis auf die Vegetation eine Abgabevorrichtung zum Ausbringen der Behandlung umfasst und so angeordnet ist, um:

das in der Vegetation vorhandene Wasser vor der Behandlung zu messen und die für die Behandlung verwendete Menge an das gemessene Wasser anzupassen; und/oder
um das in der Vegetation vorhandene Wasser vor und nach der Behandlung zu messen, um die Differenz bei der Messung des in der Vegetation vorhandenen Wassers zu berechnen, um die angewandte Behandlung zu messen.

15. Verfahren zum Betrieb einer landwirtschaftlichen Vorrichtung zur Messung von in der Vegetation vorhandenem Pflanzenwasser (115, 455, 450), wobei die genannte Vorrichtung einen Mikrowellensender mit einem Strahl umfasst, der so angeordnet ist, dass der Strahl verschiebbar ist, um durch jede einer Vielzahl von Stellen der Vegetation zu verlaufen; einen Mikrowellenempfänger; einen elektronischen Datenprozessor, der mit dem genannten Mikrowellensender und dem genannten Mikrowellenempfänger verbunden und so konfiguriert ist, dass er ein Signal von dem Mikrowellenempfänger (125, 215, 220, 520) empfängt und die Verschiebung des genannten Strahls steuert; wobei das genannte Verfahren umfasst:

Bewegen des genannten verschiebbaren Strahls des Mikrowellensender (105, 200, 500) in Richtung des Mikrowellenempfängers, um die Abschwächung des Strahls zu messen, der durch jede einer Vielzahl von Stellen der Vegetation verläuft;
Berechnung der Abschwächung des verschiebbaren Strahls zwischen Sender und Empfänger durch den elektronischen Datenprozessor, um ein räumliches 2D-Bild der Vegetation zu erhalten, wobei das Bild der Messung des in der Vegetation vorhandenen Wassers entspricht.

**Revendications**

1. Dispositif pour mesurer l'eau des plantes (115, 455, 450) présente dans la végétation comprenant :

un émetteur de micro-ondes (105, 200, 500) d'un faisceau, arrangé tel que le faisceau soit déplaçable pour passer à travers chacune d'une pluralité de localisations de la végétation ;
un récepteur de micro-ondes (125, 215, 220, 520) ;
un traiteur de données électronique raccordé audit émetteur de micro-ondes et audit récepteur de micro-ondes et configuré pour recevoir un signal à partir du récepteur de micro-ondes (125, 215, 220, 520) et pour contrôler le déplacement dudit faisceau ;
dans lequel l'émetteur de micro-ondes (105, 200, 500) est arrangé pour orienter ledit faisceau déplaçable vers le récepteur de micro-ondes pour mesurer l'atténuation du faisceau passant à travers chacune de ladite pluralité de localisations de la végétation ;

dans lequel le traiteur de données électronique est configuré pour calculer l'atténuation du faisceau déplaçable entre l'émetteur et le récepteur pour obtenir une image spatiale 2D de la végétation dans laquelle l'image correspond à la mesure de l'eau présente dans la végétation.

2. Dispositif pour mesurer l'eau des plantes (115, 455, 450) présente dans la végétation selon la revendication précédente dans lequel le récepteur de micro-ondes (125, 215, 220, 520) comprend une pluralité de récepteurs individuels.

3. Dispositif pour mesurer l'eau des plantes (115, 455, 450) présente dans la végétation selon la revendication précédente, dans lequel le récepteur de micro-ondes comprend une pluralité de récepteurs de micro-ondes individuels (125, 215, 220, 520) arrangés et distribués linéairement, tels que chaque récepteur reçoivent le faisceau passant à travers chacune de la pluralité de localisations de la végétation, en particulier la pluralité de récepteurs de micro-ondes individuels étant linéairement arrangés et distribués le long d'une direction verticale.

4. Dispositif pour mesurer l'eau des plantes (115, 455, 450) présente dans la végétation selon l'une quelconque des revendications 2-3 dans lequel l'émetteur de micro-ondes (105, 200, 500) est arrangé pour orienter ledit faisceau déplaçable vers chacun desdits récepteurs de micro-ondes individuels.

5. Dispositif pour mesurer l'eau des plantes (115, 455, 450) présente dans la végétation selon la revendication précédente dans lequel l'émetteur de micro-ondes (105, 200, 500) est arrangé pour orienter ledit faisceau déplaçable vers chacun desdits récepteurs de micro-ondes individuels (125, 215, 220, 520) un par un, tel que chaque récepteur reçoive le faisceau passant à travers chacune de ladite pluralité de localisations de la végétation.

6. Dispositif pour mesurer l'eau des plantes (115, 455, 450) présente dans la végétation selon l'une quelconque des revendications 2-5 dans lequel l'émetteur de micro-ondes comprend une antenne de faisceau orientable, en particulier une antenne de déphasage de faisceau orientable, pour orienter ledit faisceau de micro-ondes entre chacun desdits récepteurs de micro-ondes individuels (125, 215, 220, 520).

7. Dispositif pour mesurer l'eau des plantes (115, 455, 450) présente dans la végétation selon l'une quelconque des revendications 2-6 dans lequel l'émetteur de micro-ondes (105, 200, 500) comprend une pluralité d'émetteurs individuels qui sont individuellement commutables pour orienter ledit faisceau de micro-ondes entre chacun desdits récepteurs de micro-ondes individuels (125, 215, 220, 520).

8. Dispositif pour mesurer l'eau des plantes (115, 455, 450) présente dans la végétation selon la revendication précédente dans lequel l'émetteur de micro-ondes (105, 200, 500) comprend une pluralité d'émetteurs de micro-ondes individuels (105, 200, 500) arrangés et distribués linéairement tels que chaque récepteur reçoive le faisceau émis à partir de chacun desdits émetteurs passant à travers chacune de ladite pluralité de localisations de la végétation, en particulier la pluralité d'émetteurs de micro-ondes individuels étant arrangés et distribués linéairement le long d'une direction verticale.

9. Dispositif pour mesurer l'eau des plantes (115, 455, 450) présente dans la végétation selon l'une quelconque des revendications précédentes dans lequel ledit faisceau peut être déplacé par le dispositif étant mobile le long de la végétation devant être mesurée, en particulier le long d'une direction horizontale, l'émetteur de micro-ondes et le récepteur de micro-ondes étant conjointement montés dans le dispositif, l'émetteur de micro-ondes (105, 200, 500) et le récepteur de micro-ondes (125, 215, 220, 520) étant plus particulièrement conjointement montés dans des extrémités opposées d'une structure en forme d'arche.

10. Véhicule agricole (550) comprenant le dispositif pour mesurer l'eau des plantes (115, 455, 450) présente dans la végétation selon la revendication précédente dans lequel l'émetteur de micro-ondes (105, 200, 500) et le récepteur de micro-ondes (125, 215, 220, 520) sont montés dans le véhicule (550) pour se mouvoir le long de la végétation tel que le faisceau soit déplaçable pour passer à travers chacune de la pluralité de localisations de la végétation pendant que le véhicule est en mouvement.

11. Véhicule agricole (550) selon la revendication précédente comprenant un capteur de géolocalisation satellite, un capteur de géolocalisation à radiofréquence ou un capteur de déplacement pour synchroniser la mesure d'eau présente dans la végétation avec la localisation du dispositif déterminant quelle pluralité de localisations de la végétation est en train d'être mesurée alors que le véhicule (550) est en mouvement.

12. Véhicule agricole (550) selon la revendication précédente comprenant un second émetteur de micro-ondes et un second récepteur de micro-ondes monté sur le véhicule (550) pour se mouvoir le long de la végétation, tel que la seconde paire d'émetteur-récepteur mesure l'atténuation du faisceau passant à travers chacune d'une pluralité de localisations de la végétation après que la première paire d'émetteur-récepteur ait mesuré l'atténuation du faisceau passant à travers chacune de la même pluralité de localisations de la végétation.

13. Véhicule agricole (550) selon la revendication précédente, dans lequel le traiteur de données électronique est configuré pour calculer la différence dans l'atténuation du faisceau déplaçable entre la première paire d'émetteur-récepteur et la seconde paire d'émetteur-récepteur pour obtenir une image 2D correspondant à la différence dans la mesure d'eau présente dans la végétation.

14. Véhicule agricole (550) selon la revendication précédente dans lequel le dispositif pour appliquer un traitement agricole à base d'eau à la végétation comprend un distributeur pour appliquer le traitement et est arrangé pour :

mesurer l'eau présente dans la végétation avant d'appliquer le traitement et ajuster la quantité de traitement appliquée par rapport à l'eau mesurée ; et/ou
mesurer l'eau présente dans la végétation avant et après avoir appliqué le traitement pour calculer la différence dans la mesure d'eau présente dans la végétation pour mesurer le traitement appliqué.

15. Procédé de fonctionnement d'un dispositif agricole pour mesurer l'eau des plantes (115, 455, 450) présente dans la végétation, ledit dispositif comprenant un émetteur de micro-ondes d'un faisceau arrangé tel que le faisceau soit déplaçable pour passer à travers chacune d'une pluralité de localisations de la végétation ; un récepteur de micro-ondes ; un traiteur de données électronique raccordé audit émetteur de micro-ondes et audit récepteur de micro-ondes et configuré pour recevoir un signal à partir du récepteur de micro-ondes (125, 215, 220, 520) et pour contrôler le déplacement dudit faisceau ; ledit procédé comprenant :

mouvoir ledit faisceau déplaçable à partir de l'émetteur de micro-ondes (105, 200, 500) vers le récepteur de micro-ondes pour mesurer l'atténuation du faisceau passant à travers chacune de ladite pluralité de localisations de la végétation ;
calculer, à travers le traiteur de données électronique, l'atténuation du faisceau déplaçable entre l'émetteur et le récepteur pour obtenir une image spatiale 2D de la végétation dans laquelle l'image correspond à la mesure d'eau présente dans la végétation.

**Fig. 1**

**Fig. 2A**

**Fig. 2B**

EP 3 906 408 B1

**Fig. 3**

**Fig. 4**

**Fig. 5**

**Fig. 6**

**Fig. 7**

**Fig. 8(a)**

**Fig. 8(b)**

**Fig. 8(c)**

**Fig. 9(a)**

**Fig. 9(b)**

**Fig. 9(c)**

## REFERENCES CITED IN THE DESCRIPTION

**Patent documents cited in the description**

- KR 101824652

**Non-patent literature cited in the description**

- **E.R. HUNT ; BARRETT N ROCK.** Detection of changes in leaf water content using near- and middle-infrared reflectances. *Remote Sensing of Environment,* 1989, vol. 30 (1), 43-54
- **G. SCHIAVON ; D. SOLIMINI ; A. BURINI.** Sensitivity of multi-temporal high resolution polarimetric C and L-band SAR to grapes in vineyards. *2007 IEEE International Geoscience and Remote Sensing Symposium,* July 2007, 3651-3654
- Microwave Attenuation Properties of Vegetation Canopies. **FAWWAZ T ULABY et al.** IEEE TRANSACTIONS ON GEOSCIENCE AND REMOTE SENSING. IEEE SERVICE CENTER, 01 September 1985, vol. 44, 746-753
- **OGAWA Y et al.** Monitoring of water/ice state using millimeter waves for the agricultural field. *INFRARED AND MILLIMETER WAVES,* 2004
- 12TH INTERNATIONAL CONFERENCE ON TERAHERTZ ELECTRONICS, 2004. CONFERENCE DIGEST OF THE 2004 JOINT 29TH INTERNATIONAL CONFERENCE ON KARLSRUHE. IEEE, 27 September 2004, 451-452
- Microwave-band (1730MHz) accurately estimates the relative water content in poplar leaves. A comparison with a near infrared water index. **DOMINGO SANCHO-KNAPIK et al.** AGRICULTURAL AND FOREST METEOROLOGY. ELSEVIER, 27 January 2011, vol. 151, 827-832